Europäisches Patentamt

(19) European Patent Office

Office européen des brevets

(11) Numéro de publication: **0 165 141**
B1

(12) **FASCICULE DE BREVET EUROPEEN**

(45) Date de publication du fascicule du brevet:
27.09.89

(51) Int. Cl.⁴: **H 03 F 3/45**, H 03 F 3/187,
A 61 B 5/04

(21) Numéro de dépôt: 85400971.9

(22) Date de dépôt: 15.05.85

(54) Amplificateur pour signaux physiologiques.

(30) Priorité: 15.05.84 FR 8407506

(43) Date de publication de la demande:
18.12.85 Bulletin 85/51

(45) Mention de la délivrance du brevet:
27.09.89 Bulletin 89/39

(84) Etats contractants désignés:
AT BE CH DE FR GB IT LI LU NL SE

(56) Documents cités:
CH-A- 526 884
US-A- 3 818 235
US-A- 4 207 536

MEDICAL & BIOLOGICAL ENGINEERING &
COMPUTING, vol. 22, no. 1, janvier 1984, pages 77-85,
Stevenage, Herts., GB; R. VAN HEUNINGEN et al.: "A
low noise isolated amplifier system for
elecrophysiological measurements: basic
considerations and design"
IEEE TRANSACTIONS ON BIOMEDICAL ENGINEERING,
vol. BME-27, no. 12 décembre 1980, pages 699-704,
IEEE, New York, US; N.V. THAKOR et al.: "Ground-free
ECG recording with two electrodes"
IDEM
IEEE ELCTRO, vol. 7, mai 1982, pages 10/1,1-10/1,13,

(73) Titulaire: CENTRE NATIONAL DE LA RECHERCHE
SCIENTIFIQUE (CNRS), 15, Quai Anatole France,
F-75007 Paris (FR)

(72) Inventeur: Lumeau, Bernard J. F., Porte Saubotte,
F-33540 Sauveterre de Guyenne (FR)

(74) Mandataire: Joly, Jean-Jacques et al, CABINET BEAU
DE LOMENIE 55, rue d'Amsterdam, F-75008 Paris (FR)

(56) Documents cités: (suite)
New York, US; T.J. SCHWARTZ: "IC OP AMP combines
lowest noise performance available with prevision and
high speed"

**Description**

La présente invention a pour objet un amplificateur pour signaux physiologiques, c'est-à-dire des signaux tels que les potentiels évoqués, les signaux détectés pour le tracé d'électroencéphalogramme, d'électromyogramme, d'électrorétinogramme, ou encore les signaux provenant de microélectrodes. L'amplificateur objet de l'invention est donc destiné à constituer l'interface entre électrodes et appareil d'enregistrement et/ou de visualisation directe.

Les signaux physiologiques sont notamment caractérisés par une très faible amplitude, typiquement 1 μV à 1 mV, dans une bande utile allant par exemple de 0,1 Hz à 30 kHz. De plus, ces signaux sont souvent recueillis dans un fort environnement de bruit et superposés à une composante continue importante due à la polarisation des électrodes, au potentiel de membrane, au potentiel de peau, etc...

Dans les amplificateurs existants, la composante continue est généralement éliminée par le branchement d'un condensateur en série avec les électrodes. Or, une capacité en série avec l'impédance des électrodes introduit des retards qui se traduisent par des décalages sur les enregistrements de signaux prélevés. Dans le cas où ces enregistrements sont utilisés pour effectuer des mesures de latence, ou temps de réponse, l'on conçoit que les déphasages introduits par l'amplificateur peuvent gravement affecter l'exactitude des mesures.

Le document Medical & Biological Engineering & Computing, Vol. 22, No 1, janvier 1984 pages 77–85 décrit un amplificateur pour signaux physiologiques comportant un étage d'amplification d'entrée de tye différentiel ayant deux entrées reliées à deux électrodes, un circuit d'alimentation de l'étage d'entrée ayant un point de masse flottant et deux sorties à des potentiels situés de part et d'autre de celui du point de masse flottant, un étage d'amplification intermédiaire ayant un circuit de contre-réaction pour éliminer la composante continue du signal recueilli, un étage d'amplification de sortie et un filtre passe-bas destiné à éliminer les composantes à hautes fréquences du signal recueilli. Avec un tel amplificateur connu, des déphasages sont introduits par les moyens de filtrage et l'on retrouve les inconvénients précités.

Aussi, la présente invention a-t-elle pour but de fournir un amplificateur pour signaux physiologiques n'introduisant pas de variation sensible du temps de propagation de groupe par l'élimination de la composante continue et le filtrage des hautes fréquences. L'invention a aussi pour but de fournir un amplificateur ayant un très faible niveau de bruit et dans lequel des moyens sont prévus pour éviter toute réinjection de courant dans les électrodes.

Ce but est atteint grâce à un amplificateur du type comportant:
- un étage d'amplification d'entrée de type différentiel ayant deux entrées destinées à être reliées respectivement à une première et une deuxième électrodes, et deux sorties,
- un circuit d'alimentation dudit étage d'amplification d'entrée, ayant un point de masse flottant et deux sorties qui sont à des potentiels situés de part et d'autre du potentiel de référence défini par le point de masse flottant,
- un étage d'amplification intermédiaire ayant deux entrées différentielles reliées aux sorties de l'étage d'entrée et une sortie reliée à l'une de ses entrées par un circuit de contreréaction pour former filtre passe-haut afin d'éliminer la composante continue du signal recueilli,
- un étage d'amplification de sortie, et
- un filtre passe-bas branché en série avec l'étage d'amplification de sortie afin d'éliminer les composantes à hautes fréquences du signal recueilli, amplificateur dans lequel, conformément à l'invention:
- le point de masse flottant (MF) du circuit d'alimentation est fixé par rapport à la tension de mode commun définie à partir des tensions d'entrée de l'étage d'entrée,
- un circuit correcteur de phase est branché en série avec l'étage d'amplification intermédiaire pour corriger le déphasage introduit par celui-ci et ne pas introduire de variation de temps de propagation de groupe, et
- le filtre passe-bas branché en série avec l'étage d'amplification de sortie est choisi de manière à ne pas introduire de variation de temps de propagation de groupe.

Une première caractéristique de l'amplificateur conforme à l'invention consiste dans l'élimination de la composante continue et des composantes hautes fréquences sans introduire de retard variable en sortie. Pour la composante continue, ce résultat est obtenu grâce à un étage d'amplification avec contreréaction associé à un circuit correcteur de phase et, pour les composantes hautes fréquences, grâce un filtre passe-bas ayant un temps de propagation de groupe régulier dans la bande passante tel que, par exemple un filtre de Bessel.

Une autre caractéristique consiste dans la création d'un potentiel de référence formant masse flottante et représentatif du potentiel du point milieu entre les entrées de l'amplificateur. De la sorte, on réalise une réjection du mode commun, ce qui augmente l'immunité aux bruits parasites.

De préférence, une source de courant est branchée sur chaque entrée de l'étage d'entrée de manière à compenser les courants de fuite réinjectés sur les électrodes. Il est ainsi possible de limiter ces courants de fuite à de très faibles valeurs, notamment inférieurs à 10 pA.

De préférence encore, un circuit de protection est prévu qui comprend un élément à seuil destiné à fournir un signal de commande de coupure d'alimentation lorsque la tension sur l'une quelconque des sorties du circuit d'alimentation devient, en valeur absolue, inférieure à un seuil donné. De la sorte, une protection des entrées est réalisée vis-à-vis d'une coupure d'alimentation, ou vis-à-vis d'un déséquilibre important du potentiel de référence tel qu'il peut résulter de la mise en l'air d'une électrode.

En vue de l'utilisation de l'amplificateur conforme à l'invention pour la transmission à un appareil d'enregistrement et/ou visualisation de signaux recueillis sur un patinet, il peut être souhaité de découpler la «masse» de l'appareil de celle du patient. Dans ce cas, l'amplificateur et l'appareil sont isolés électriquement l'un de l'autre, et des moyens de transmission du signal sont prévus en sortie de l'amplificateur pour pouvoir appliquer à l'appareil un signal représentatif du signal de sortie de l'amplificateur. Avantageusement, ces moyens de transmission comportent un premier coupleur électro-optique dont l'émetteur est relié à l'étage de sortie de l'amplificateur, un second coupleur électro-optique dont l'émetteur est relié à une borne destinée à être connectée à l'appareil, et un circuit d'asservissement ayant des entrées reliées aux récepteurs des coupleurs électro-optiques et une sortie reliée à l'émetteur du second coupleur pour asservir le signal appliqué à l'émetteur du second coupleur au signal appliqué à l'émetteur du premier coupleur.

D'autres particularités et avantages de l'amplificateur selon l'invention ressortiront à la lecture de la description faite ci-après, à titre indicatif mais non limitatif, en référence aux dessins annexés sur lesquels:
- la figure 1 est un schéma général par blocs d'un mode de réalisation d'un amplificateur conforme à l'invention,
- la figure 2 est un schéma détaillé de l'étage d'entrée de l'amplificateur de la figure 1,
- la figure 3 est un schéma détaillé de l'étage d'amplification intermédiaire, formant étage d'élimination de la composante continue et du circuit associé de correction de phase,
- la figure 4 est un schéma détaillé de l'étage d'amplification de sortie, du filtre des composantes hautes fréquences et du circuit de sortie de l'amplificateur de la figure 1,
- la figure 5 est un schéma détaillé du circuit d'alimentation de l'amplificateur de la figure 1,
- la figure 6 est un schéma détaillé d'une variante de réalisation de l'étage d'entrée de l'amplificateur, et
- la figure 7 est un schéma détaillé d'une variante de réaslition du circuit de sortie de l'amplificateur.

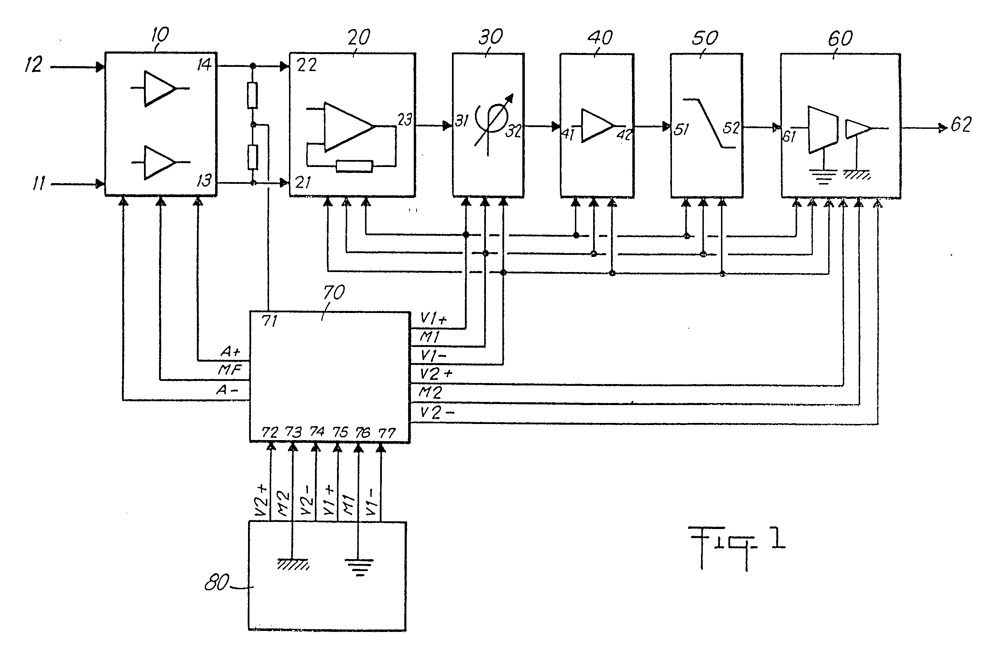

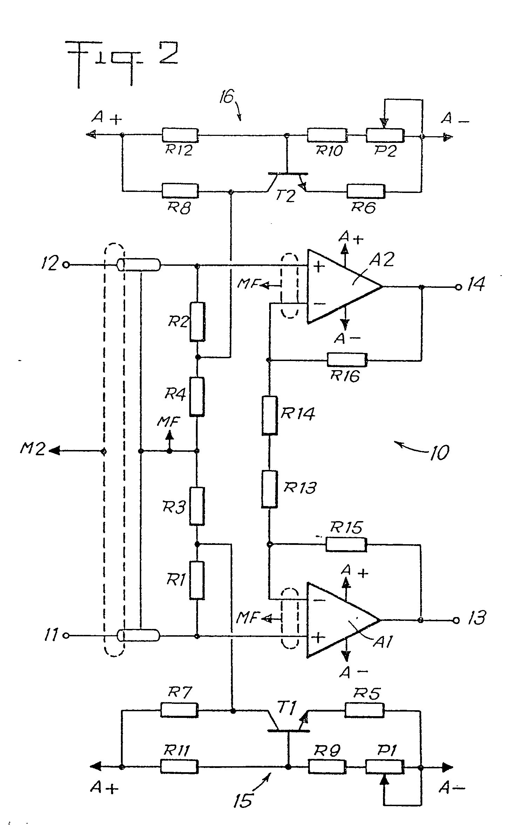

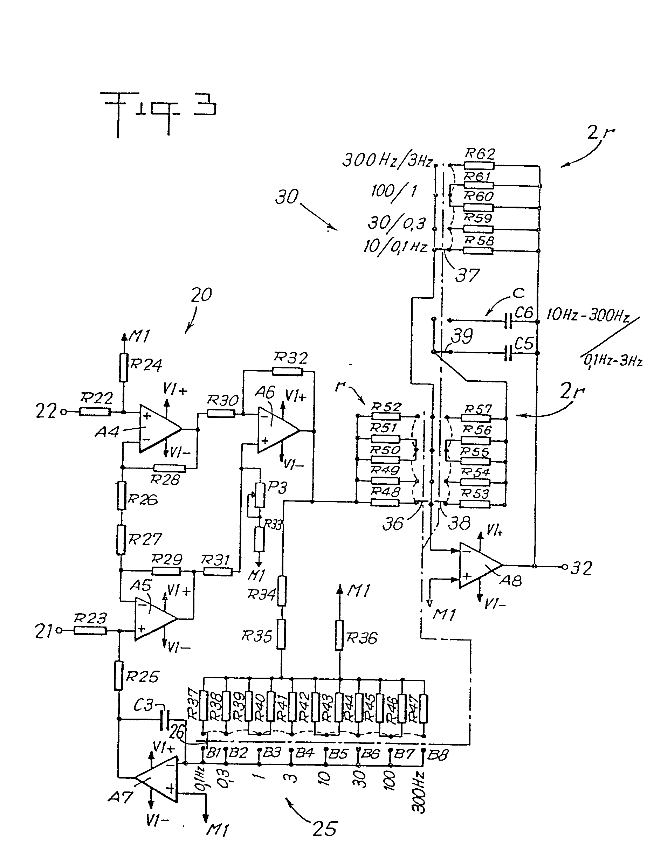

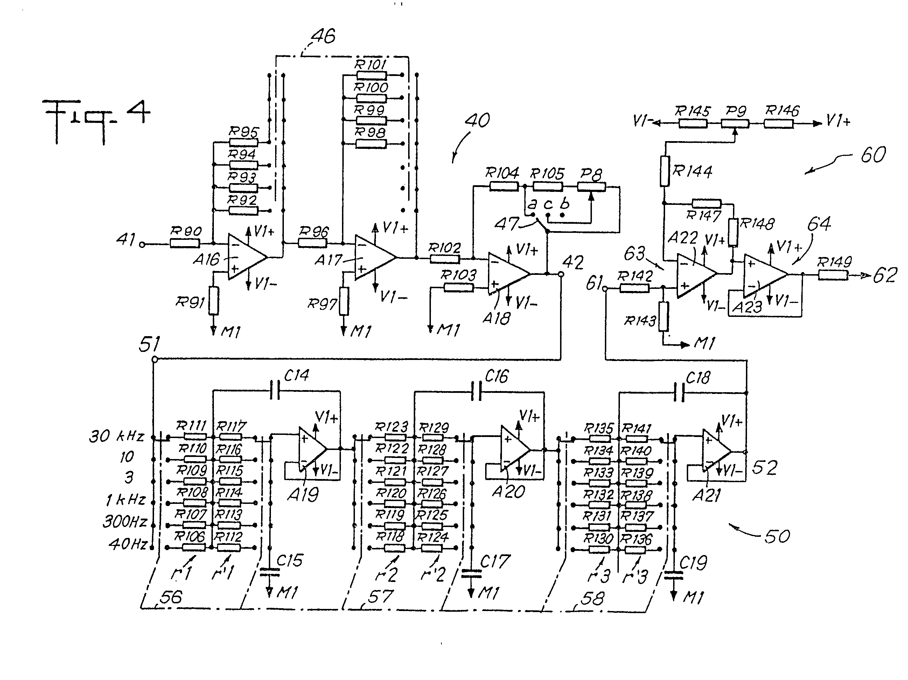

L'amplificateur conforme à l'invention, tel que représenté sur la figure 1, comprend un étage d'amplification 10 d'entrée, un étage 20 d'amplification intermédiaire formant filtre passe-haut, un circuit 30 correcteur de déphasage, un étage d'amplification de sortie 40, un filtre passe-bas 50, un circuit de sortie 60 et un circuit d'alimentation 70.

L'étage d'amplification d'entrée 10 a deux entrées différentielles 11, 12 sur lesquelles il reçoit les signaux provenant de deux électrodes (non représentées). Après une première amplification, ces signaux sont disponibles sur deux sorties différentielles 13, 14 de l'étage d'entrée.

L'étage d'amplification intermédiaire 20 a deux entrées différentielles 21, 22 qui sont connectées aux sorties 13, 14 de l'étage d'entrée, et fournit, sur sa sortie 23 un signal correspondant, après amplification et élimination de la composante continue, à la différence entre les signaux reçus sur les entrées 21, 22.

Le circuit 30 correcteur de déphasage a une entrée 31 reliée à la sortie de l'étage intermédiaire et a pour fonction de compenser le déphasage variable en fonction de la fréquence introduit par ce dernier.

La sortie 32 du circuit correcteur de phase est reliée à l'entrée 41 de l'étage 40 d'amplification de sortie qui détermine, avec les étages 10 et 20 le gain total de l'amplificateur.

Le filtre passe-bas 50 a son entrée reliée à la sortie 42 de l'étage 40 afin d'éliminer les composantes hautes fréquences. On notera que le filtre passe-bas pourrait être branché devant l'étage 40 plutôt que derrière celui-ci.

Le circuit de sortie 60 a son entrée 61 reliée à la sortie 52 du filtre 50 et délivre sur sa sortie 62 le signal de sortie de l'amplificateur. Le circuit 60 remplit une fonction d'adaptation pour le raccordement de l'amplificateur à un appareil (non représenté) destiné à l'enregistrement et/ou la visualisation des signaux amplifiés.

Enfin, un circuit d'alimentation 70 fournit les potentiels de référence et d'alimentation nécessaires au fonctionnement des circuits de l'amplificateur. Le circuit 70 a une entrée 71 connectée au point milieu entre les sorties 13 et 14 de l'étage d'entrée afin d'élaborer un potentiel de référence, ou masse flottante MF, image du potentiel du point milieu entre les entrées 11 et 12. Une alimentation 80 fournit deux tensions V1+ et V1− symétriques par rapport à une masse M1 ainsi que deux tensions V2+ et V2− symétriques par rapport à une masse M2. Le circuit d'alimentation 70 a des entrées 72 à 77 reliées respectivement aux sorties V2+, M2, V2−, V1+, M1 et V1 de l'alimentation stabilisée 80. Il fournit aux circuits 10 la masse flottante MF et deux tensions d'alimentation A+ et A− symétriques par rapport à la masse flottante MF. Le circuit 70 transmet par ailleurs d'une part, aux circuits 20, 30, 40, 50 et à une partie d'entrée du circuit 60 la masse M1 et les tensions V1+, V1−, et d'autre part, à une partie de sortie du circuit 60, la masse M2 et les tensions V2+ et V2−. Des moyens de sécurité sont prévus dans le circuit d'alimentation pour protéger les entrées de l'amplificateur vis-à-vis des tensions d'alimentation.

Lorsque la masse du patient est confondue avec la masse électrique, les masses M1 et M2 sont identiques, de même que V1+ et V2+, et V1− et V2−.

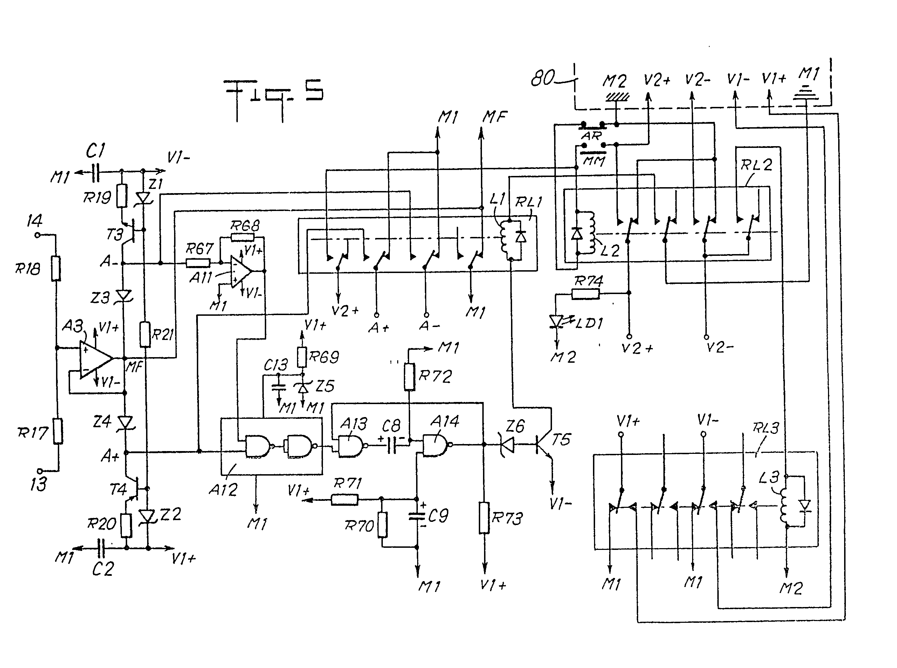

On se reportera maintenant à la figure 2 qui illustre de façon détaillée l'étage d'entrée 10.

Les signaux provenant des électrodes sont acheminés aux entrées 11, 12 au moyen de câbles à double blindage, le blindage extérieur étant à la masse patient et le blindage intérieur étant connecté à la masse flottante MF.

Les entrées 11 et 12 sont reliées aux entrées positives de deux amplificateurs opérationnels respectifs A1 et A2 alimentés sous A+ et A–. Ce sont des composants de type bipolaire choisis pour leur très faible niveau de bruit. Ces amplificateurs ont des courants de fuite qu'il est préférable de combattre pour limiter à de très faibles valeurs les intensités des courants réinjectés dans les électrodes. A cet effet, deux sources de courant 15, 16 sont prévues pour compenser les courants de fuite sur les entrées positives des amplificateurs opérationnels respectifs A1 et A2.

Quatre résistances R1, R3, R4 et R2 sont branchées en série entre les entrées 11 et 12 et les courants produits par les sources 15, 16 sont injectés respectivement au point commun entre R1 et R3 au point commun entre R2 et R4. Le point commun entre R3 et R4 est relié à la masse flottante MF.

Chaque source 15, 16 comprend un transistor à jonction T1, T2 du même type que les jonctions des amplificateurs opérationnels A1, A2 afin de compenser automatiquement les dérives d'origine thermique. L'émetteur du transistor T1 (T2) est relié à la borne A– par une résistance R5 (R6) et son collecteur est relié a la borne A+ par une résistance R7 (R8). La base du transistor T1 (T2) est reliée à un diviseur de potentiel comprenant un potentiomètre P1 (P2) et deux résistances R9, R11 (R10, R12) branchées en série entre les bornes A– et A+, la base du transistor T1 (T2) étant connectée au point commun entre R9 et R11 (R10 et R12). Le courant produit par chaque source 15, 16 est ajusté au moyen du potentiomètre P1, P2 afin de compenser le courant de fuite de l'amplificateur opérationnel associé. Il est ainsi possible de limiter les courants de fuite à des valeurs inférieures à 10 pA.

Les entrées négatives des amplificateurs A1 et A2 sont reliées, d'une part, entre elles par deux résistances R13, R14 branchées en série et, d'autre part, aux sorties respectives des amplificateurs A1, A2 par deux résistances respectives R15, R16. Les résistances R13, R14, R15 et R16 déterminent le gain G1 de l'étage 10:

G1 = 1 + (R15 + R16)/(R13 + R14).

Des valeurs numériques ou références des éléments des circuits décrits sont données à titre d'exemple à la fin de la description. Avec les valeurs choisies pour R13 à R16, le gain G1 est égal à 10.

Il est à noter que l'étage 10 comprend en fait deux voies identiques l'une entre l'entrée 11 et la sortie 13, l'autre entre l'entrée 12 et la sortie 14. Par ailleurs, les composants A1 et A2 ayant été choisis pour leur très faible niveau de bruit, il est nécessaire de choisir pour les résistances R13, R14, R15 et R16 des composants précis et de qualité pour ne pas réintroduire de bruit par leur intermédiaire.

La figure 3 illustre l'étage 20 d'amplification intermédiaire et d'élimination de la composante continue, et le circuit 30 de correction de phase.

Les entrées différentielles 21, 22 sont reliées par des résistances R23, R22 aux entrées positives de deux amplificateurs opérationnels respectifs A5, A4. L'entrée positive de l'amplificateur A4 est connectée à la masse M1 par une résistance R24. Les entrées négatives des amplificateurs A4, A5 sont reliées, d'une part entre elles par deux résistances R26, R27 branchées en série et, d'autre part aux sorties respectives de ces amplificateurs par deux résistances respectives R28, R29. Les sorties des amplificateurs A4, A5 sont en outre reliées respectivement à l'entrée négative et à l'entrée positive d'un amplificateur opérationnel A6 par des résistances R30 et R31. Une résistance R32 connecte la sortie de l'amplificateur A6 à son entrée négative tandis qu'une résistance R33 en série avec un poteniomètre P3 connecte l'entrée positive de l'amplificateur A6 à la masse M1. Le potentiomètre P3 permet un réglage optimal du taux de réjection de mode commun.

La sortie de l'amplificateur opérationnel A6 forme la sortie de l'étage 20. Un circuit de contre-réaction 25 connecte cette sortie à l'entrée positive de l'amplificateur opérationnel A5. Le circuit 25 comprend trois résistances R34, R35 et R36 branchées en série entre l'amplificateur A6 et la masse M1, le point commun de R35 et R36 étant relié à un ensemble de résistances R37 à R47. Les résistances R37 à R47 peuvent être connectées sélectivement à des bornes respectives B1 à B8 au moyen d'interrupteurs groupés par exemple dans un commutateur rotatif 26. Plus précisément, les bornes B1 à B8 permettent la mise en parallèle à la résistance R37, de la résistance R38, des résistances R39 et R40 branchées en parallèle, de la résistance R41, des résistances R42 et R43 branchées en parallèle, de la résistance R44, des résistances R45 et R46 branchées en parallèle et de la résistance R47. La liaison entre la résistance R37 et la borne B1 est fixe, le commutateur 26 assurant la mise en parallèle sur R37 des autres résistances R38 à R47 de façon successive et cumulative lorsqu'il est déplacé des positions B1 à B8. Les bornes B1 à B8 sont reliées en commun à l'entrée négative d'un amplificateur opérationnel A7 dont la sortie est reliée à l'entrée positive de l'amplificateur A5 par une résistance R25. Un condensateur C5 relie l'entrée négative de l'amplificateur A7 à sa sortie tandis que l'entrée positive de l'amplificateur A7 est reliée à la masse M1. L'amplificateur A7 est alimenté sous V1+ et V1–, de même que A4, A5 et A6.

En boucle ouverte, l'étage 20 forme un étage d'amplification dont le gain est:

$$G2 = \frac{R32}{R30}\left(1 + \frac{R28 + R29}{R26 + R27}\right)\frac{R24 + R25}{R22 + R23 + R24 + R25} = G \times \frac{R24 + R25}{R22 + R23 + R24 + R25}$$

soit G2 = 100 avec les valeurs données en annexe.

En boucle fermée, la fonction de transfert de l'étage 20

$$G2\,(p) = \frac{G}{1 + \beta \cdot G/T \cdot p} \cdot \frac{R24 + R25}{R22 + R23 + R24 + R25}, \text{avec}$$

$$\beta = \frac{R23}{R23 + R25} \cdot \frac{R36}{R34 + R35 + R36} \text{ et } T = C5 \cdot Rk$$

où Rk est la résistance branchée entre le point commun des résistances R35 et R36, et l'entrée négative de l'amplificateur A7.

Ainsi, l'étage 20 constitue un filtre passe-haut dont la fréquence de coupure dépend de Rk. Avec les valeurs données en annexe, les positions B1 à B8 correspondent à des fréquences de coupure fcb égales respectivement à 0,1 Hz, 0,3 Hz, 1 Hz, 3 Hz, 10 Hz, 30 Hz, 100 Hz et 300 Hz. Le diviseur R23/(R23 + R25) dans le circuit de contre-réaction détermine la limite maximale admissible du niveau de composante continue correspondant à la saturation de l'amplificateur A7; dans l'exemple donné, cette limite est de 2,5 V en entrée de l'étage 20, soit 250 mV en entrée de l'amplificateur. Une telle valeur n'est normalement pas atteinte par suite de la polarisation des électrodes, du potentiel de membrane, du potentiel de peau, ... Si toutefois ce seuil maximal est dépassé, l'étage d'entrée peut être remplacé par celui décrit plus loin en référence à la figure 6 dont le branchement sur les entrées 21, 22 déconnecte automatiquement le circuit 10.

L'étage amplificateur contre-réactionné 20 permet ainsi une élimination de la composante continue sans branchement de capacité en série avec les électrodes, mais il introduit toutefois un déphasage. Dans le cas où le commutateur 26 est positionné sur B1 (fcb = 0,1 Hz), le retard introduit varie de 1250 ms à une fréquence de 0,1 Hz à 159 μs à une fréquence de 10 Hz.

Le circuit 30 permet de corriger ce déphasage dans une large mesure. Il comporte un amplificateur opérationnel A8 alimenté sous V1− et V1+, dont l'entrée négative est reliée à la sortie de l'amplificateur A6 par une résistance de valeur r et dont l'entrée positive est reliée à la masse M1. Le circuit de réaction entre la sortie et l'entrée négative de l'amplificateur A8 est formé par une première résistance de valeur 2r branchée en parallèle avec le circuit série formé par une deuxième résistance de valeur 2r et un condensateur de capacité c.

Selon la position d'un commutateur 36, la résistance d'entrée r est constituée par une résistance R48, ou la mise en parallèle de la résistance R48 avec, cumulativement, une résistance R49 deux résistances R50 et R51 branchées en parallèle, et une résistance R52. De façon correspondante, et selon la position d'un commutateur 37, la première résistance 2r est constituée par une résistance R58 ou la mise en parallèle de R58 avec, cumulativement, une résistance R59, deux résistances R60 et R61 branchées en parallèle, et une résistance R62. Toujours de façon correspondante, et selon la position d'un commutateur 38, la deuxième résistance 2r est constituée par une résistance R53 ou la mise en parallèle de R53 avec, cumulativement, une résistance R54, deux résistances R55 et R56 branchées en parallèle, et une résistance R57.

Enfin, selon la position d'un commutateur 39, le condensateur de capacité c est constitué par un condensateur C5 ou un condensateur C6. Les quatre positions des commutateurs 36, 37, 38 correspondent respectivement aux fréquences de coupure 0,1 Hz, 0,3 Hz, 1 Hz et 3 Hz lorsque le condensateur C5 est en circuit et respectivement aux fréquences de coupure 10 Hz, 30 Hz, 100 Hz et 300 Hz lorsque le condensateur C6 est en circuit.

La fonction de transfert du circuit 30 est:

$$G3(p) = -\frac{2\,(1 + T3.p)}{1 + 2\,T3.p} \text{ avec } T3 = 2.rc$$

Les commutateurs 36, 37 et 38 sont couplés mécaniquement entre eux et, avantageusement, au commutateur 26 pour être actionnés simultanément au moyen d'un même organe de réglage. Le basculement du commutateur 39 peut être provoqué automatiquement lors du retour des commutateurs 26, 36, 37 et 38 à leur première position. De la sorte, les valeurs r, 2r et c sont sélectionnées automatiquement pour correspondre au réglage de la fréquence de coupure fcb, les déphasages introduits par l'étage 20 variant en fonction de fcb. A titre indicatif, dans la position correspondant à fcb = 0,1 Hz, la correction apportée par le circuit 30 permet, dans la bande 3 Hz–20 kHz, de ramener le retard maximum de 1,76 ms (sans corrections) à 60 μs (avec correction).

L'étage amplificateur de sortie 40 est branché directement en sortie du circuit correcteur 30.

Dans l'exemple illustré figure 4, il est formé par trois amplificateurs opérationnels A16, A17, A18 branchés en cascade et alimentés sous V1− et V1+. Le premier A16 a son entrée négative reliée au circuit 30 par une résistance R90 et son entrée positive reliée à la masse M1 par une résistance R91. Une résistance R92, R93, R94 ou R95 est branchée entre la sortie de l'amplificateur A16 et son entrée négative selon qu'un commutateur 46 à sept positions est respectivement dans sa première, sa deuxième, sa troisième ou l'une quelconque de ses quatrième à septième positions. Le second amplificateur A17 a son entrée négative reliée à la sortie de l'amplificateur A16 par une résistance R96 et son entrée positive reliée à la masse M1 par une résistance R97. Une résistance R98, R99, R100 ou R101 est branchée entre la sortie de l'amplificateur A17 et son entrée négative selon que le commutateur 46 est respectivement dans l'une quelconque de ses première à quatrième positions, sa cinquième, sa sixième ou sa septième position. Le troisième amplificateur A18 a son entrée négative reliée à la sortie de l'amplificateur A17 par une résistance R102 et son entrée positive reliée à la masse M1 par une résistance R103. Deux résistances R104 et R105 et un potentiomètre P8 sont branchés en série entre la sortie de l'amplificateur A18 à trois points a, b, c respectivement connecté au point commun entre les résistances R104 et R105, connecté au curseur du potentiomètre P8 et en l'air.

En choisissant des résistances R92 à R95 valant respectivement 1 fois, 2 fois, 5 fois et 10 fois la résistance R90 et, de la même façon, des résistances R98 à R101 valant respectivement 1 fois, 2 fois, 5 fois et 10 fois la résistance R96, le commutateur 46 permet ainsi de donner au gain combiné $G'4$ des amplificateurs A16 et A17 les valeurs 1, 2, 5, 10, 20, 50 et 100. En donnant par ailleurs aux résistances R104 et R102 la même valeur, et à la somme des résistances R104, R105, P8 une valeur 10 fois égale à celle de la résistance R102, le commutateur 47 permet de donner au gain $G''4$ de l'amplificateur A18 la valeur 1, la valeur 10 ou une valeur variant entre 1 et 10. De la sorte, il est possible d'ajuster dans une très large mesure le gain G4 de l'étage amplificateur 40 entre les valeurs 1 et 1000, d'où, pour l'amplificateur, dans son ensemble, un gain réglable en étage, entre $10^3$ et $10^6$.

Les composantes hautes fréquences du signal de sortie de l'étage 40 sont filtrées au moyen du filtre passe-bas 50 représenté figure 4. Ce dernier est réalisé par un filtre de Bessel car un tel filtre permet d'introduire un temps de propagation de groupe fixe. La réalisation d'un tel filtre est bien connue en soi. Pour obtenir une coupure assez franche, et de très faibles variations sur le retard, il est souhaitable de réaliser un filtre de Bessel d'ordre relativement élevé, par exemple du 6e ordre. Ainsi, le filtre 50 est formé de trois étages branchés en cascade. Le premier comprend un amplificateur opérationnel A19 dont l'entrée positive est reliée à la sortie de l'étage d'amplification 40 par deux résistances de valeurs r1, r'1 branchées en série, et est reliée à la masse M1 par un condensateur C15. La sortie de l'amplificateur A19 est, d'une part, reliée directement à son entrée négative et, d'autre part, reliée par un condensateur C14 au point commun entre les résistances de valeurs r1 et r'1. Un second amplificateur opérationnel A20 a son entrée positive reliée à la sortie de l'amplificateur A19 par deux résistances de valeurs r2, r'2 branchées en série, et à la masse M1 par un condensateur C17. La sortie de l'amplificateur A20 est, d'une part, reliée directement à son entrée négative et, d'autre part, reliée par un condensateur C16 au point commun entre les résistances de valeurs r2 et r'2. Un troisième amplificateur opérationnel A21 a son entrée positive reliée à la sortie de l'amplificateur A20 par deux résistances de valeurs r3, r'3 branchées en série, et à la masse M1 par un condensateur C19. La sortie de l'amplificateur A21 est, d'une part, reliée directement à son entrée négative et, d'autre part, reliée par un condensateur C18 au point commun entre les résistances de valeurs r3 et r'3.

Les résistances de valeurs r1 et r'1 sont sélectionnées au moyen d'un commutateur 56 à six positions parmi des résistances R106 à R111 et R112 à R117. De la même façon, un commutateur 57 permet de sélectionner les résistances de valeurs r2 et r'2 parmi des résistances R118 à R123 et R124 à R129 et un commutateur 58 permet de sélectionner la résistances de valeur r3 et r'3 parmi les résistances R130 à R135 et R136 à R141. Les commutateurs 56, 57 et 58 sont couplés mécaniquement pour pouvoir être actionnés par un même organe de réglage, la position des commutateurs correspondant à une fréquence de coupure fch particulière du filtre passe-bas. A titre indicatif, les valeurs des résistances R106 à R141 et des condensateurs C14 à C19 sont choisies (voir en annexe) pour permettre de sélectionner des fréquences de coupures fch égales à 40 Hz, 300 Hz, 1 kHz, 3kHz, 10 kHz et 30 kHz.

La figure 4 montre également le circuit de sortie 60 qui comporte un premier étage 63 de rattrapage de dérive de continu et un deuxième étage suiveur 64 pour permettre de sortir sur une impédance type. Le premier étage 63 comprend un amplificateur opérationnel A22 dont l'entrée positive est reliée à la sortie du filtre passe-bas 50 par une résistance R142 et à la masse M1 par une résistance R143 et dont l'entrée négative est reliée par une résistance 144 au curseur d'un potentiomètre P9. Ce dernier est lié, d'un côté, à la borne V1– par une résistance R145 et, de l'autre côté, à la borne V1+ par une résistance R146. Des résistances R148 et R147 sont branchées en série entre la sortie de l'amplificateur A22 et son entrée négative. Le réglage de la tension du curseur du potentiomètre P9 permet de compenser en sortie de l'amplificateur une éventuelle dérive de continu.

Enfin, l'étage suiveur 64 est formé par un amplificateur opérationnel A23 dont l'entrée positive est reliée à la sortie de l'amplificateur A22, et dont la sortie est, d'une part reliée directement à l'entrée négative et, d'autre part reliée à la borne de sortie 62 de l'amplificateur par une résistance R149 dont la valeur est choisie de façon typique égale à 50 Ω. Les amplificateurs A22 et A23, de même que A19 à A21 sont alimentés sous V1– et V1+.

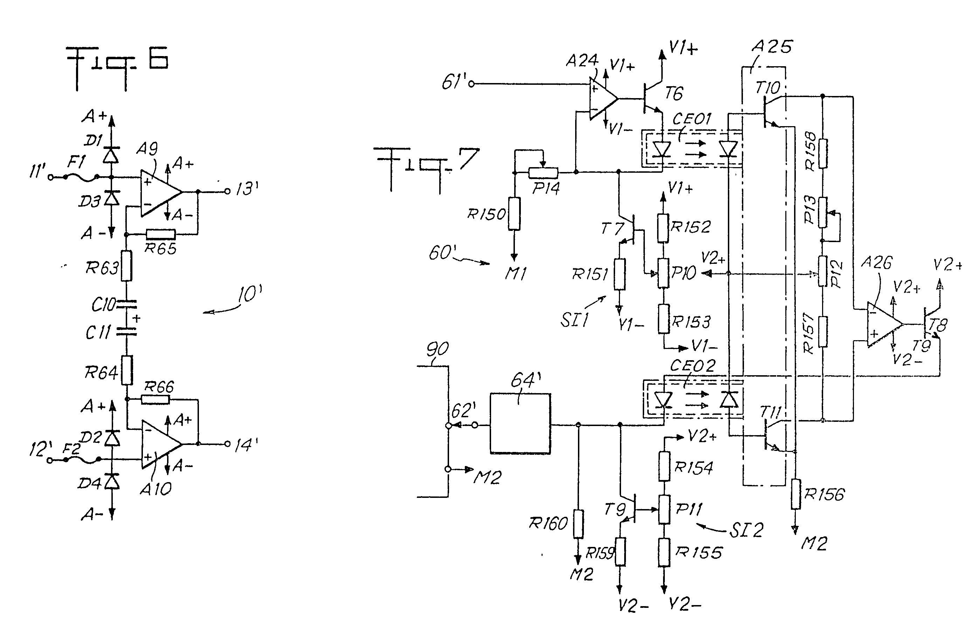

On se reportera maintenant à la figure 5 qui illustre le circuit d'alimentation 70.

Celui-ci comporte d'abord un circuit d'élaboration de la masse flottante MF et de tensions A+ et A– symétriques par rapport à la masse flottante MF. Ce circuit comprend un amplificateur opérationnel A3 dont l'entrée positive est reliée au point commun entre deux résistances égales R17 et R18 branchées en série entre les sorties 13 et 14 du circuit d'amplification d'entrée 10. L'amplificateur A3 a son entrée négative reliée directement à sa sortie en constitue ainsi un étage suiveur délivrant sur sa sortie un signal qui suit les évolutions du signal de mode commun d'entrée. La sortie de l'amplificateur A3 constitue la borne de potentiel de référence MF, celui-ci étant l'image du potentiel du point milieu entre les sorties (donc entre les entrées) du circuit d'entrée 10. Afin de reproduire fidèlement le potentiel de ce point milieu, on choisira des résistances R17 et R18 de précision.

Deux diodes Zener Z3, Z4 relient la borne MF respectivement au collecteur d'un transistor T3 et au collecteur d'un transistor T4. Ces transistors ont leurs émetteurs reliés respectivement aux bornes V1– et V1+ par des résistances R19 et R20 et leurs bases reliées à ces mêmes bornes par des diodes Zener Z1 et Z2. Une résistance R21 servant à la polarisation de Z1 et Z2 est branchée entre les bases des transistors T1 et T2. Des condensateurs C1 et C2 sont branchés entre la masse M1 et, respectivement, les bornes V1– et V1+. Les tensions A– et A+ symétriques par rapport à la masse MF sont disponibles sur les collecteurs des transistors T1 et T2.

A titre indicatif, les bornes V1– et V1+ sont respectivement à –12 V et +12 V par rapport à la masse M1 et les bornes A– et A+ sont respectivement à –8,2 V et +8,2 V par rapport à la masse MF. Les diodes Zener Z3 et Z4 sont alors des diodes 8,2 V tandis que les diodes Zener Z1 et Z2 sont des diodes 2,4 V.

Le circuit d'alimentation 70 comprend également des moyens de détection de variations anormales de A+ et A– pour éviter qu'apparaissent sur les entrées de l'amplificateur des tensions continues injustifiées.

Ces moyens de détection comportent un comparateur formé par un circuit A12 en technologie MOS réalisant la fonction ET et dont le seuil est fixé par la tension qui l'alimente. Un amplificateur opérationnel A11 reçoit sur son entrée négative la tension A– par l'intermédiaire d'une résistance R67. Cet amplificateur A11 a son entrée positive reliée à la masse M1 et sa sortie reliée à son entrée négative par une résistance R68 égale à R67; il fonctionne donc en inverseur pour transmettre l'inverse de A– sur une première entrée de la porte A12, la tension A+ étant directement transmise sur une deuxième entrée de cette porte. La borne d'alimentation du circuit A12 est reliée au point commun entre une diode Zener Z5 et une résistance R69 branchées en série entre la masse M1 et V1+. Un condensateur C13 est connecté entre ce même point commun et la masse M1. La tension appliquée sur la borne d'alimentation de A12 est choisie grâce à la diode Z5 (par exemple 10 V) pour régler le seuil de la porte A12 à la valeur voulue (par example 4 V).

Ainsi, dès que A+ ou A– devient en valeur absolue inférieure à un seuil prédéterminé par déplacement de MF d'une valeur supérieure au seuil (référencé par rapport à M1), la sortie de la porte A12 passe au niveau logique bas. Inversement, tant que A+ et A– restent en valeur absolue au-dessus de ce seuil, la sortie de la porte A12 reste au niveau logique haut.

La sortie de la porte A12 est reliée à une entrée d'une porte A13 dont la sortie est reliée par l'intermédiaire d'un condensateur C8 à une entrée d'une autre porte A14. Les portes A13 et A14 réalisent chacune la fonction NON-ET. La sortie de la porte A14 est reliée à une deuxième entrée de la porte A13, tandis qu'une deuxième entrée de la porte A14 est reliée au point milieu d'un diviseur de potentiel formé par deux résistances R70 et R71 branchées en série entre M1 et V1+, ce diviseur étant destiné à appliquer une tension au niveau logique haut sur la deuxième entrée de A14. Un condensateur C9 est branché entre cette deuxième entrée et la masse M1 tandis qu'une résistance R72 est branchée entre la première entrée de la porte A14 et la masse M1.

La sortie de la porte A14 est reliée par une résistance R73 à V1+ et, par une diode Zener Z6, à la base d'un transistor T5 dont l'émetteur est au potentiel V1– et dont le collecteur est relié à une extrémité de la bobine d'excitation L1 d'un relais RL1. Ce dernier a quatre contacts mobiles. Le premier de ces contacts est relié à V2+ et est mobile entre une position de repos inactive et une position de travail dans laquelle il connecte V2+ à une première borne d'un interrupteur de mise en marche MM et à une borne d'une bobine d'excitation L2 d'un relais RL2 dont il assure l'automaintien. Le deuxième contact mobile du relais RL1 est mobile entre une position de repos, dans laquelle il relie la borne de sortie A+ à la masse M1, et une position de travail, dans laquelle il ferme l'alimentation A+ assurant ainsi l'alimentation positive de l'étage amplificateur d'entrée 10. De façon similaire, le troisième contact mobile du relais RL1 est mobile entre une position de repose, dans laquelle il relie la borne de sortie A– à la masse M1, et une position de travail, dans laquelle il ferme l'alimentation A– assurant ainsi l'alimentation négative de l'étage 10. Enfin, le quatrième contact mobile du relais RL1 est mobile entre une position de repos dans laquelle il relie MF à M1 et une position de travail dans laquelle il découple les masses MF et M1.

Le deuxième relais RL2 a sa bobine d'excitation L2 branchée entre la première borne de l'interrupteur de mise en marche MM et une première borne d'un interrupteur d'arrêt AR. Le relais RL2 a quatre contacts mobiles. Le premier de ces contacts est relié à la borne de sortie V2+ et est mobile entre une position de repos, dans laquelle il relie la borne de sortie V2+ à la masse M2, et une position de travail, dans laquelle il relie la borne V2+ à la borne correspondante V2+ de l'alimentation 80. Le deuxième contact mobile du relais RL2 est connecté à la masse M1 et est mobile entre une position de repos inactive et une position de travail dans laquelle il relie la borne M1 à l'autre extrémité de la bobine L1. Le troisième contact mobile du relais RL2 est relié à la borne de sortie V2– et est mobile entre une position de repos, dans laquelle il relie la borne V2– à la masse M2, et une position de travail dans laquelle il relie la borne V2– à la borne correspondante V2– de l'alimentation 80. Le quatrième contact mobile du relais RL2 est relié également à la borne de sortie V2– et est mobile entre une position de repos inactive et une position de travail dans laquelle il relie cette borne V2– à une bobine d'excitation L3 d'un relais RL3. L'autre extrémité de la bobine L3 est reliée à la masse M2. Le relais RL3 a quatre contacts mobiles dont deux sont utilisés et sont reliés respectivement aux bornes de sorties V1+ et V1–. Au repos, les contacts mobiles relient ces bornes de sortie à M1 tandis que, en position de travail, les contacts mobiles relient les bornes V1+ et V1– aux bornes correspondantes de l'alimentation 80.

Enfin, les deuxièmes bornes des interrupteurs AR et MM sont reliées respectivement à la masse M2 et à la borne V2+ de l'alimentation 80.

Le fonctionnement est le suivant.

Les interrupteurs MM et AR constituent un même composant fonctionnant par impulsion directive (par exemple une impulsion vers le haut met en marche, une impulsion vers le bas le coupe). Lorsqu'une action est fournie sur MM, la bobine L2 est alimentée et les contacts mobiles du relais RL2 viennent en position de travail, validant les tensions V2+ et V2–. La venue du quatrième contact mobile du relais RL2 en position de travail assure l'alimentation du relais RL3, validant les tensions V1+ et V1–. Dès l'établissement de V1+, la sortie de A14 passe au niveau haut, débloquant T5. Le deuxième contact mobile du relais RL2 assure alors l'alimentation de la bobine L1 du relais RL1 qui, par son premier contact, assure, l'auto-maintien de RL2 une fois l'action relâchée. Les tensions A+ et A– et MF sont élaborées. Tant qu'elles sont supérieures en valeur absolue au seuil fixé, le transistor T5 est débloqué et les contacts mobiles du relais RL1 en position de travail, valident les bornes A+, A– en sortie du circuit d'alimentation 70. Si l'une des tensions A+ et A– devient en valeur absolue inférieure au seuil fixé, un niveau logique haut est appliqué à la première entrée de A14 à travers C8; le transistor T5 est bloqué le relais RL1 revient au repos entrainant les retours au repos des relais RL2 et RL3. Les circuits ne sont plus alimentés, et la borne MF est connectée à la borne M1. Tout risque de réinjection de courant à l'entrée par suite d'un fort déséquilibre dû par exemple à la mise en l'air d'une des électrodes saturant l'étage d'entrée, ou à la coupure d'une des alimentations V1– ou V1+ est ainsi évité.

Le circuit à constante de temps C8–R72 permet de maitenir la sortie de A14 au niveau bas, et le transistor T5 bloqué, jusqu'à ouverture effective des contacts de RL2, évitant ainsi une possible oscillation si l'équilibre à l'entrée de A12, suite à l'ouverture de RL1, est retrouvé avant coupure de l'alimentation de L1.

Lorsqu'une action est fournie sur AR, le circuit d'alimentation de la bobine L2 est coupé, ce qui fait retomber le relais RL2 et, par conséquent, les relais RL1 et RL3.

On notera qu'un témoin lumineux sous forme d'une diode électro-luminescente LD1 est branché en série avec une résistance R74 entre les bornes M2 et V2+. On a envisagé ci-avant un fonctionnement avec isolement entre les alimentations M1, V1+, V1– et M2, V2+, V2–. Un fonctionnement sans isolement est possible, M1 et M2 étant confondues, de même que V1+ et V2+, et V1– et V2–. Toutes les alimentations sont prises en sortie de RL2. La constante de temps du circuit C9–R70 permet alors, si une tension A+ ou A– devient défectueuse, de maintenir le transistor T5 bloqué jusqu'à ouverture complète des contacts de RL2.

L'amplificateur conforme à l'invention qui vient d'être décrit est particulièrement remarquable par son très faible niveau de bruit (le bruit ramené à l'entrée, sans électrode et avec entrées à la masse est inférieur à 0,35 µV efficace dans la bande 0,1 Hz à 3 kHz) et son très faible courant de fuite (inférieur à 10 pA dans la configuration de la figure 2 et à 1 pA avec l'entrée répondant à la figure 6 décrite ci-après). Il présente de plus un taux de réjection de mode commun supérieur à 120 dB. Sans électrodes, la résistance d'entrée est de 10 mΩ en différentiel et la capacité d'entrée est d'environ 5 pF; avec la configuration de la figure 6, ces valeurs passent - $10^{13}$ Ω et 1 pF.

La figure 6 illustre une variante de réalisation de l'étage d'amplification d'entrée, utilisable notamment en cas de présence d'une forte composante continue ou d'une forte impédance d'entrée (microélectrodes par exemple).

Les entrées 11' et 12' sont reliées aux entrées positives de deux amplificateurs opérationnels respectifs A9, A10 par l'intermédiaire de fusibles F1, F2. Les entrées négatives des amplificateurs A9, A10 sont, d'une part, reliées aux sorties de ces amplificateurs par des résistances respectives R65, R66 et, d'autre part, reliées entre elles par un circuit série comprenant une résistance R63, deux condensateurs polarisés C10, C11 et une résistance R64. Les deux amplificateurs sont alimentés sous A– et A+.

Le gain de cet étage d'entrée 10' est de la forme:

$$G'1 = 1 + \frac{r'4/r4}{1 + 1/r4 \cdot c4 \cdot p}$$

avec r'4 = R65 = R66, r4 = R63 = R64 et c4 = C10 = C11.

Ainsi, le gain pour la composante continue est égal à 1, ce qui permet, compte-tenu des valeurs indiquées plus haut, de tolérer une composante continue de 2,5 V. Par contre, le gain pour les composantes non continues reste supérieur, par exemple égal à 10. Les valeurs des composants sont choisies de manière que le fréquence de coupure soit très faible afin de limiter au minimum le retard qu'introduit cet étage d'entrée dans la bande utile. A titre d'exemple, avec r4 = 11,1 kΩ, r'4 = 100 kΩ et c4 = 2200 µF, le retard maximum total de l'amplificateur est de 175 µs dans la bande 3 Hz–20 kHz, au lieu de 60 µs dans l'exemple de réalisation précédent (pour fcb = 0,1 Hz et fch = 30 kHz).

L'étage d'entrée 10' peut s'avérer utile lorsque l'amplificateur est connecté à des microélectrodes ayant une forte tendance à la polarisation. Les canaux de ces microélectrodes utilisées en iontophorèse sont parfois obstrués et il est courant de les déboucher par application d'une tension relativement forte. Afin d'éviter une destruction d'au moins l'étage d'entrée si cette opération est réalisée par mégarde sans débranchement de l'amplificateur, les entrées positives des amplificateurs A9, A10 sont reliées à la borne A+ par des diodes respectives D1, D2 et à la borne A- par des diodes respectives D3, D4. Ces diodes sont choisies pour leur faible courant de fuite. Une protection supplémentaire est fournie par les fusibles F1 et F2.

La figure 7 illustre une variante de réalisation du circuit de sortie dans le cas où la masse M1 utilisée dans l'amplificateur et la masse M2 de l'appareil 90 d'enregistrement et/ou visualisation connecté en sortie de l'amplificateur sont différentes (fonctionnement avec isolement).

Le circuit de sortie 60' a son entrée 61' reliée à la sortie 52 du circuit de sortie 50. Cette entrée est reliée à l'entrée positive d'un amplificateur opérationnel A24 dont l'entrée négative est reliée à la masse M1 par une résistance variable formée par un potentiomètre P14 en série avec une résistance R150. La sortie de l'amplificateur A24 est reliée à la base d'un transistor T6 dont le collecteur est relié à la borne V1+ et dont l'émetteur est relié à l'entrée négative de l'amplificateur A24 par l'intermédiaire de l'émetteur d'un coupleur électro-optique CEO1 dont le récepteur est branché entre la basse d'un transistor T10 et la borne V2+. Un second coupleur électro-optique CEO2 a son récepteur branché entre la base d'un transistor T11 et la borne V2+. Les émetteurs des transistors T10 et T11 sont reliés à la masse M2 par une résistance commune R156, et leurs collecteurs sont reliés entre eux par un circuit série formé par une résistance R158, deux potentiomètres P13, P12 et une résistance R157, le curseur du potentiomètre P12 étant relié à V2+. Ce circuit série permet de régler la polarisation des transistors T10, T11. L'ensemble CEO1, T10, CEO2, T11 constitue un seul circuit A25. Un amplificateur opérationnel A26 a ses entrées négative et positive connectées respectivement aux collecteurs des transistors T10 et T11 et sa sortie reliée à la base d'un transistor T8 dont l'émetteur est à la borne V2+ et dont le collecteur est relié à la masse M2 par l'intermédiaire de l'émetteur du coupleur CEO2 et d'une résistance R160.

Une source de courant SI1 est branchée entre la borne V1- et le point commun au potentiomètre P14 et à l'émetteur du coupleur CEO1. La source SI1 comprend un transistor T7 dont le collecteur est relié au potentiomètre P14 et dont l'émetteur et relié à la borne V1- par une résistance R151. La base due transistor T7 est reliée au curseur d'un potentiomètre P10 branché en série entre des résistances R152 et R153 entre les bornes V1+ et V1-. Une source de courant SI2 est branchée entre la borne V2- et le point commun à la résistance R160 et à l'émetteur du coupleur CEO2. La source SI2 comprend un transistor T11 dont le collecteur est relié à la résistance R160 et dont l'émetteur est relié par une résistance R159 à une borne d'alimentation V2-. La base du transistor T9 est reliée au curseur d'un potentiomètre P11 branché en série entre des résistances R154 et R155 entre les bornes V2+ et V2-. Le point commun de la résistance R160 et du collecteur de T9 est relié à la sortie 62' du circuit 60' par un étage suiveur 64' identique à l'étage 64 du circuit 60.

Le circuit 60' qui vient d'être décrit fonctionne comme un circuit d'asservissement, l'amplificateur A26 imposant le courant de sortie de manière que les signaux sur ses entrées soient égaux, d'où égalité entre les courants traversant les émetteurs des deux coupleurs. L'on reproduit ainsi fidèlement sur la sortie 62' le signal reçu à l'entrée 61', le réglage étant effectué au moyen notamment des potentiomètres P12 et P14.

Tableau 1: valeurs des éléments d'amplification d'entrée 10 (figure 2) - les résistances sont données en ohms.

R1, R2: 100 · 10^6
R3, R4, R5, R6: 10^3
R7, R8: 1,82 · 10^3
R9: 24,9
R10: 24,9
R11, R12: 10^3
R13, R14: 111
R15, R16: 10^3
P1, P2: 500
A1, A2: OP 27 FZ
T1, T2: 2 N 2222 A

Tableau 2: valeurs des éléments de l'étage d'amplification intermédiaire contre-réactionné 20 (figure 3) - les résistances sont données en ohms.

R22, R23: 332
R24, R25: 1,27 · 10^3
R26: 23,6
R27: 147
R28, R29, R30, R31: 10^3
R32: 10 · 10^3
R33: 8.25 · 10^3
R34: 464
R35: 24,9
R36: 24,9
R37: 402 · 10^3
R38, R39: 205 · 10^3
R40: 80,6 · 10^3
R41, R42: 20,5 · 10^3
R43: 8,06 · 10^3
R44, R45: 2,05 · 10^3
R46: 806
R47: 162
P3: 2 · 10^3
C3: 2 µF
A4, A5, A6: OP 27 GZ
A7: OPA 111 BM

Tableau 3: valeurs des éléments du circuit 30 correcteur de déphasage (figure 3) – les résistances sont données en ohms.
R48: $162 \cdot 10^3$
R49, R50: $82,5 \cdot 10^3$
R51: $32,4 \cdot 10^3$
R52: $8,25 \cdot 10^3$
R53, R58: $324 \cdot 10^3$
R54, R55, R59, R60: $162 \cdot 10^3$
R56, R61: $63,4 \cdot 10^3$
R57, R62: $16,2 \cdot 10^3$
C5: 1 µF
C6: 10 nF
A8: OPA 111 BN

Tableau 4: valeurs des éléments du filtre passe bas 50 (figure 4) – les résistances sont données en ohms.
R106, R112, R118, R124, R130, R136: $750 \cdot 10^3$
R107, R113, R119, R125, R131, R137: $100 \cdot 10^3$
R108, R114, R120, R126, R132, R138: $30,1 \cdot 10^3$
R109, R115, R121, R127, R133, R139: $10 \cdot 10^3$
R110, R116, R122, R128, R134, R140: $3,01 \cdot 10^3$
R111, R117, R123, R129, R135, R141: $10^3$
C14: 3,38 nF
C15: 3,24 nF
C16: 3,84 nF
C17: 2,57 nF
C18: 5,7 nF
C19: 1,36 nF
A19, A20, A21: TDB 156 DP

**Revendications**

1. Amplificateur pour signaux physiologiques, comportant:
– un étage d'amplification d'entrée (10) de type différentiel ayant deux entrées destinées à être reliées respectivement à une première et une deuxième électrodes, et deux sorties,
– un circuit d'alimentation (70) de l'étage d'amplification d'entrée (10), ayant un point de masse flottant et deux sorties (A+, A–) qui sont à des potentiels situés de part et d'autre du potentiel de référence (MF) défini par le point de masse flottant,
– un étage d'amplification intermédiaire (20) ayant deux entrées différentielles reliées aux sorties de l'étage d'entrée et une sortie reliée à l'une de ses entrées par un circuit de contreréaction pour former filtre passe-haut afin d'éliminer la composante continue du signal recueilli,
– un étage d'amplification de sortie (40), et
– un filtre passe-bas (50) branché en série avec l'étage d'amplification de sortie (40) afin d'éliminer les composantes à hautes fréquences du signal recueilli, ledit amplificateur étant caractérisé en ce que:
– le point de masse flottant (MF) du circuit d'alimentation est fixé par rapport à la tension de mode commun définie à partir des tensions d'entrée de l'étage d'entrée (10),
– un circuit (30) correcteur de phase est branché en série avec l'étage (20) d'amplification intermédiaire pour corriger le déphasage introduit par celui-ci et ne pas introduire de variation de temps de propagation de groupe, et
– le filtre passe-bas (50) branché en série avec l'étage d'amplification de sortie (40) est choisi de manière à ne pas introduire de variation de temps de propagation de groupe.

2. Amplificateur selon la revendication 1, caractérisé en ce qu'une source de courant (15, 16) est branchée sur chaque entrée de l'étage d'entrée (10) de manière à compenser les courants de fuite réinjectés sur les électrodes.

3. Amplificateur selon l'une quelconque des revendications 1 et 2, caractérisé en ce que le filtre passe-bas (50) est un filtre de Bessel.

4. Amplificateur selon l'une quelconque des revendications 1 à 3, caractérisé en ce qu'il comporte un circuit de protection comprenant un élément à seuil (A12) destiné à fournir un signal de coupure d'alimentation lorsque la tension (A+, A–) sur l'une quelconque desdites sorties du circuit d'alimentation (70) devient, en valeur absolue, inférieure à un seuil donné.

5. Amplificateur selon l'une quelconque des revendications 1 à 4, caractérisé en ce qu'il comporte un circuit de sortie (60') avec un premier coupleur électro-optique (CEO1) dont l'émetteur est relié à l'étage de sortie de l'amplificateur, un second coupleur électro-optique (CEO2) dont l'émetteur est relié à une borne destinée à être connectée à un appareil d'enregistrement et/ou de visualisation isolé de l'amplificateur, et un comparateur (A26) ayant des entrées reliées aux récepteurs des coupleurs électro-optiques et une sortie reliée à l'émetteur du second coupleur pour asservir l'amplitude du signal appliqué à l'émetteur du second coupleur à l'amplitude du signal appliqué à l'émetteur du premier coupleur.

6. Amplificateur selon la revendication 1, caractérisé en ce que le circuit d'amplification d'entrée (10') présente un gain plus élevé pour les composantes non continues que pour les composantes continues des signaux reçus sur ses entrées de manière à faciliter l'élimination desdites composantes continues par l'étage d'amplification intermédiaire.

**Patentansprüche**

1. Verstärker für physiologische Signale, umfassend:
– einen Eingangsverstärker (10) vom Differentialtyp mit zwei Eingängen, die dazu bestimmt sind, an jeweils eine erste bzw. eine zweite Elektrode gelegt zu werden und mit zwei Ausgängen
– einen Versorgungskreis (70) für den Eingangsverstärker (10) mit gleitendem Massenpunkt und zwei Ausgängen (A+, A–), die ein Potential aufweisen, das jeweils auf einer Seite des Referenzpotentials (MF), das durch den gleitenden Massenpunkt definiert wird, liegen,
– einen Zwischenverstärker (20), der zwei differentielle Eingänge aufweist, die mit den Ausgängen der Eingangsstufe verbunden sind und einen Ausgang, der mit einem der Eingänge durch einen Gegenreaktionskreis verbunden ist, um ein Hochpassfilter zu bilden, um den Gleichstromanteil des empfangenen Signals zu eliminieren,
- einen Ausgangsverstärker (40) und
- ein Tiefpassfilter (50), das in Serie mit dem Ausgangsverstärker (40) geschaltet ist, um die hohen Frequenzen des empfangenen Signals zu eliminieren, der Verstärker ist dadurch gekennzeichnet, dass:
- der fliessende Massenpunkt (MF) des Versorgungskreises fix bezüglich der Durchschnittsspannung ist, die ausgehend von den Eingangsspannungen der Eingangsstufe (10) definiert ist,
- ein Phasenkorrekturkreis (30) in serie mit dem Zwischenverstärker (20) geschalten ist, um die durch ihn eingebrachte Phasenverschiebung zu korrigieren und um keine veränderliche Zeitverschiebung dieser Gruppe einzubringen und dadurch,
- dass das Niederpassfilter (50) in Serie mit dem Ausgangsverstärker (40) geschalten ist und so ausgelegt ist, dass es keine Veränderung der Zeitverschiebung dieser Gruppe hervorruft.

2. Verstärker nach Anspruch 1, dadurch gekennzeichnet, dass eine Stromquelle (15, 16) auf jeden Eingang des Eingangsverstärkers (10) so gelegt ist, dass sie die auf die Elektroden zurückgeleiteten Streuströme kompensiert.

3. Verstärker nach einem der Ansprüche 1 und 2, dadurch gekennzeichnet, dass das Tiefpassfilter (50) ein Besselfilter ist.

4. Verstärker nach einem der Ansprüche 1 bis 3, dadurch gekennzeichnet, dass er einen Schutzkreis umfasst, der ein Schwellenelement (A12) aufweist, das dazu bestimmt ist, ein Signal zum Abschalten der Versorgung zu liefern, wenn die Spannung (A+, A−) an einem der Ausgänge des Versorgungskreises (70) in Absolutwerten kleiner als eine vorgegebene Schranke ist.

5. Verstärker nach einem der Ansprüche 1 bis 4, dadurch gekennzeichnet, dass er einen Ausgangskreis (60′) mit einem ersten elektrooptischen Kuppler (CEO1), dessen Emitter mit dem Verstärkerausgang verbunden ist und einem zweiten elektrooptischen Kuppler (CEO2), dessen Emitter an einer Klemme liegt, die dazu bestimmt ist, mit einem Speicher und/oder Anzeigeinstrument verbunden zu werden, welches vom Verstärker getrennt ist, aufweist, und dass ein Vergleicher (A26) Eingänge aufweist, die mit den Aufnehmern der elektrooptischen Kuppler verbunden sind und einen Ausgang, der mit dem Emitter des zweiten Kupplers verbunden ist, um die Amplitude des am Emitter des zweiten Kupplers liegenden Signals der Amplitude des am Emitter des ersten Kupplers liegenden Signals zu unterwerfen.

6. Verstärker nach Anspruch 1, dadurch gekennzeichnet, dass der Eingangsverstärkerkreis (10′) für die Wechselstromanteile eine höhere Verstärkungsrate aufweist als für die Gleichstromanteile der an seinen Eingängen ankommenden Signale, um so die Eliminierung der Gleichstromanteile durch den Zwischenverstärkerkreis zu erleichtern.

## Claims

1. Amplifier for physiological signals, comprising:
- a differential-type input amplification stage (10) having two inputs intended to be connected respectively to a first and a second electrodes, and two outputs,
- a feed circut (70) for the input amplification stage (10), having a floating mass point and two outputs (A+, A−) which are at potentials situated to either side of the reference potential (MF) defined by the floating mass point, and two outputs (A+, A−) which are at potentials situated to either side of the reference potential (MF) defined by the floating mass point,
- an intermediate amplification stage (20) having two differential inputs connected to the outputs of the input stage and an output connected to one of its inputs by a reverse feedback circuit to form a highpass filter in order to remove the continuous component of the collected signal,
- an output amplification stage (40), and
- a low-pass filter (50) connected in series with the output amplification stage (40) in order to remove the high-frequency components of the collected signal, said amplifier being characterised as follows:
- the floating mass point (MF) of the feed circuit is fixed in relation to the common mode voltage defined from the input voltages of the input stage (10),
- a phase-correcting circuit (30) is connected in series with the intermediate amplification stage (20) to correct the phase displacement introduced by the latter and not introduce any group propagation time variation, and
- the low-pass filter (50) connected in series with the output amplification stage (40) is selected so as not to introduce any group propagation time variation.

2. Amplifier according to claim 1, characterised in that a current source (15, 16) is connected to each input of the input stage (10) so as to compensate the leak currents fed back at the electrodes.

3. Amplifier according to either of claims 1 and 2, characterised in that the low-phase filter (50) is a Bessel filter.

4. Amplifier according to any one of claims 1 to 3, characterised in that it includes a protective circuit comprising a threshold element (A12) intended to supply a supply cutoff signal whenever the voltage (A+, A−) at any of said outputs of the feed circuit (70) becomes, in absolute value, lower than a given threshold.

5. Amplifier according to any one of claims 1 to 4, characterised in that it includes an output circuit (60′) with a first electro-optical coupler (CEO1) of which the emitter is connected to the output stage of the amplifier, a second electro-optical coupler (CEO2) of which the emitter is connected to a terminal intended to be connected to a recording and/or display device isolated from the amplifier, and a comparator (A26) having inputs connected to the receivers of the electro-optical couplers and an output connected to the emitter of the second coupler to subject the amplitude of the signal applied to the emitter of the second coupler to the amplitude of the signal applied to the emitter of the first coupler.

6. Amplifier according to claim 1, characterised in that the input amplification circuit (10') presents a higher amplification factor for the non-continuous components than for the continuous components of the signals received at its inputs, so as to facilitate the removal of said continuous components by the intermediate amplification stage.

Fig. 1

Fig. 2

Fig 3

Fig-4

Fig. 5

EP 0 165 141 B1

Fig. 7

Fig. 6